# EUROPEAN PATENT SPECIFICATION

(11) **EP 2 260 851 B1**
(45) Date of publication and mention of the grant of the patent: **13.04.2016**
(21) Application number: 09724203.6
(22) Date of filing: 12.02.2009
(51) Int. Cl.: A61K 45/06

(54) **OLEANOLIC ACID FOR THE TREATMENT OF MULTIPLE SCLEROSIS**
OLEANSÄURE ZUR BEHANDLUNG VON MULTIPLER SKLEROSE
L'ACIDE OLÉANOLIQUE DESTINÉ AU TRAITEMENT DE LA SCLÉROSE EN PLAQUES

(30) Priority: 28.03.2008 ES 200800873
(43) Date of publication of application: 15.12.2010
(73) Proprietor: Consejo Superior De Investigaciones Científicas, 28006 Madrid (ES)
(72) Inventor: NIETO CALLEJO, Maria Luisa, 47003 Valladolid (ES); MARTÍN MONTAÑA, Rubén, 47003 Valladolid (ES); CARVALHO TAVARES, Juliana, 47003 Valladolid (ES); RUIZ GUTIÉRREZ, Valentina, 41012 Sevilla (ES)
(74) Representative: Pons Ariño, Angel
(86) International application number: PCT/ES2009/070024
(87) International publication number: WO 2009/118441

(56) References cited:
- WO-A1-98/51302
- WO-A1-99/65478
- WO-A1-2009/129546
- MAIA, J. L. ET AL.: 'Oleanolic acid, a pentacyclic triterpene attenuates capsaicin- induced nociception in mice: Possible mechanisms' PHARMACOLOGICAL RESEARCH vol. 54, 2006, pages 282 - 286, XP024907827
- OVESNA, Z. ET AL.: 'Pentacyclic triterpenoic acids: new chemoprotective compounds' NEOPLASMA vol. 51, no. 5, 2004, pages 327 - 333, XP008141621

## Description

The present invention contemplates a pharmacological composition comprising as an active ingredient oleanolic acid, for the prophylaxis and treatment of multiple sclerosis. The invention demonstrates the ability of this natural compound, present in the cuticle of olives and olive leaves, as well as in oils where these fractions have a significant presence (olive-pomace oil), to reduce markedly the clinical signs of experimental autoimmune encephalomyelitis, referred to herein as EAE. This action is associated with an increase of both weight and survival of experimental animals as well as a reduction of the inflammatory reaction and cerebrovascular permeability. It is therefore an object of this invention to provide a pharmaceutical and/or nutraceutical composition with applications for treating Multiple Sclerosis (hereafter MS).

### BACKGROUND OF THE INVENTION

Multiple Sclerosis is an autoimmune, inflammatory and degenerative disease of the central nervous system (CNS) directed against myelin proteins of the brain and spinal cord. It is considered as one of the major neurological illness of young adults, but although more common in women, the severity of the disease is more pronounced in male carriers. MS is characterized by the presence of plaques or lesions of demyelination in the white matter of the CNS, resulting in an abnormal nerve conduction that mainly affects muscle control. Signs and symptoms vary widely, depending on the location of the lesions. Thus, i) motor symptoms may include speech impairment, weakness, tremors and difficulty walking, ii) sensory symptoms may include numbness, tingling, pain and visual disturbances and iii) the psychological symptoms can include changes in mood, and depression. In most patients, the disease occurs during or after a previous stage of relapses and remissions, while in a small percentage of patients (10-15%), the evolution of the disease is progressive from the beginning. The attacks lead to a continuous process of demyelination and remyelination, which causes scarring of nerve fibbers and a progressive disability. Although the exact cause of the disease is still unknown, several studies have supported the hypothesis of a viral aetiology, but none of the viruses studied has emerged as the causative agent searched. So, current theories suggest that the etiology of MS may be related to a combination of autoimmune, environmental and viral factors that act together with an underlying genetic predisposition.

Experimental autoimmune encephalomyelitis (EAE) induced in susceptible strains of animals provides the best available model for understanding events in MS and to test new drugs that could lead to novel therapies (Raine et al. Lab Invest. 1980, 43: 150-7). In this model, immunization of the CNS is achieved by injection with specific antigens (myelin basic protein, proteolipid protein or myelin/oligodendrocyte glycoprotein) and adjuvants, which induce a T cell-mediated attack on the brain and spinal cord (Pettinelli and McFarlin, J. Immunol. 1981,127: 1420-1423).. Thus, in the CNS of animals with EAE are observed perivascular and parenchymal inflammatory infiltrates consisting mainly of (CD8⁺ and CD4⁺) T cells, B cells and activated macrophages that have crossed the blood-brain barrier (BBB), due to alterations in its permeability. Subsequently, it takes place the activation of resident cells such as microglia and astrocytes (Schonrock LM. et al Neuropathol Appl Neurobiol. 1998, 24: 320-30). Depending on the species and/or animal strain as well as on the antigen used, the inflammatory lesions may be accompanied, or not, by areas of demyelination. One of the characteristic features of EAE is the progressive weight loss during the clinical phase of the disease, which is rapidly reverted when the animals recover (Ruuls et al, J. Immunology, 1996, 157:5721-5731). The recovery is also associated with the production of cytokines such as IL-10 and TGF-(Kennedy et al., J. Immunol. 1992, 149:2496-2505).

The currently available treatments for multiple sclerosis are intended to suppress the immune-inflammatory component of the disease. At present, the disorder is treated symptomatically by administration of high-dose of glucocorticoids at the onset of acute neurological symptoms. However, due to the numerous and serious side effects of steroids, it is not possible to carry out continuous preventive therapy. Some patients also received immunosuppressive agents, although they are often poorly tolerated. Therefore it is necessary to identify new treatments for MS to resolve the above mentioned issues and to minimize or slow the progression of the disease.

Thus, pharmacological research is focused on finding novel therapeutic agents, and in recent years the plant kingdom is proving to be an excellent resource for finding these new compounds. Even, it have been developed several lines of research that seek to analyze the beneficial effects of natural compounds present in vegetables, in order to relate the composition of the diet with the development/modulation of immune-inflammatory diseases, to define possible nutritional therapeutic strategies. In this context, oleanolic acid is a pentacyclic triterpenoid of oleanane group frequently present in plants used in traditional medicine in different countries. It comes in the form of free acid or as triterpenoid saponins aglycones (Liu J., J Ethnopharmacol 1995, 49: 57-68), and has been isolated from various plant species, including *Olea europaea.*

The literature since 1906 (Canzoneri F., Gazz chim ital, 1906, 36:372) recognize that the oleanolic acid as a permanent constituent of olive leaves and fruits. In the olive tree, is mainly in leaves, green fruits, especially in the cuticle, and in the residual liquor from the dressing olives. It is also found in mature olives, olive oil and pomace oil.

There are numerous publications on the therapeutic properties of triterpenes and in particular there are multiple studies that collect the biological and pharmacological activities of the oleanolic acid. These include: hepatoprotective (Liu Y et al., Toxicology Letters 1998, 95: 77-85), inflammatory (Mañez S et al. Eur J Pharmacol. 1999, 334: 103-5; Marquez-Martin A et al. Cytokines. 2006), antitumoral (R. Martin et al. Cancer Res 2007, 67: 3741-51, John ME et al. J Nutr. 2006, 136: 2553-7), anti-HIV (Zhu YM et al. , Bioorg Med Chem Lett. 2001, 11: 3115-8; Kashiwara Y et al., J Nat Prod 1998, 61: 1090-5), vasodilatory (R. Rodriguez-Rodriguez et al. Br J Nutr. 2004, 92 : 635-42), hypoglycaemic (H. Sato et al. Biochem Biophys Res Commun. 2007, 362: 793-8, Yoshikawa M et al. Biofactors. 2000, 13: 231-7), and lipid lowering activities (BL Ma.Traditional Medicine and Pharmacology. 1986, 2: 28-29).

The present invention relates to the search for new treatments for MS and describes a new pharmacological application of the oleanolic acid, as an agent that markedly reduces the clinical and immuno-inflammatory hallmarks of the experimental autoimmune encephalomyelitis.

### SUMMARY OF THE INVENTION

### Brief Description

The present invention describes oleanolic acid for the prevention and/or treatment of multiple sclerosis. Oleanolic acid (3β-hydroxyolean-12-en-28-oic acid) has the following formula:

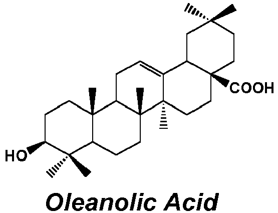

The invention also discloses a pharmaceutical composition for use in the treatment of multiple sclerosis comprising a therapeutically effective amount of oleanolic acid, together with, optionally, one or more adjuvants and/or pharmaceutically acceptable vehicles.

### Detailed Description

This invention pertains to a composition comprising oleanolic acid as the agent that reduces the clinical and the immuno-inflammatory signs observed in the experimental autoimmune encephalomyelitis induced in C57BL/6 mice (female 6-8 weeks of age), animal model of multiple sclerosis, and that in addition significantly delays the onset of the disease, in particular (see Example 1):
a) Daily ip administration of 6 mg/kg of oleanolic acid (OA1) decreases the severity of the disease, and
b) Daily ip administration of 6 mg/kg of oleanolic acid (OA2) delays the onset of EAE.

These actions are manifested through: i) amelioration neurological symptoms, ii) weight gain, iii) decrease of cellular and molecular extravasation, iv) decrease expression of key proteins in inflammatory processes, v) increased survival of EAE mice.

Therefore, the results suggest that this triterpene may have protective effects on the BBB integrity and on the inflammatory events related to the development of EAE, which could lead to an improvement of the clinical symptoms associated with MS, in the EAE model, as well as other neurodegenerative disorders. Furthermore, since this product is a natural substance that can be isolated from olives, this compound could be used as a supplement to the diet or in nutraceutical preparations.

Thus, an object of this invention is the use of oleanolic acid in the development of pharmaceutical compositions for the prevention and/or treatment of multiple sclerosis.

In this invention the term "oleanolic acid" also includes its pharmaceutically acceptable salts and solvates.

Another object of this invention is a pharmaceutical composition for the treatment of multiple sclerosis, hereafter "pharmaceutical composition of this invention", comprising a therapeutically effective amount of oleanolic acid, together with, optionally, one or more adjuvants and or a pharmaceutically acceptable vehicles.

In this disclosure, the term "pharmaceutically acceptable vehicle" refers to those substances, or combination of substances, known in the pharmaceutical industry, used in the preparation of pharmaceutical forms and includes adjuvants, solids or liquids, solvents or surfactants.

The pharmaceutical composition may also contain one or more therapeutic agents that will eventually enhance the therapeutic action of the pentacyclic triterpene compound or increase their spectrum of activity.

The oleanolic acid will be present in the pharmaceutical composition in a therapeutically effective amount, i.e. in an appropriate amount to exert its therapeutic effect. In a particular embodiment, the pharmaceutical composition provided by this invention, contains between 0.01% and 99.99% by weight, of oleanolic acid, and may be available at any appropriate dispensation form according to the administration route chosen, eg oral, parenteral, intraperitoneal or topical. A review of the different pharmaceutical forms of drug administration and preparation procedures can be found, for example, in the Treaty of Galenic Pharmacy, C. Faulí i Trillo, 1st edition, 1993, Luzán 5, SA Editions.

### BRIEF DESCRIPTION OF THE DRAWINGS

**Figure 1****:** Effect on body weight (A), and on clinical signs (B) due to the progression of the disease effected by oleanolic injected daily after first symptoms. OA1
**Figure 2****:** Effect on body weight (A), and on clinical signs (B) due to the progression of the disease effected by oleanolic injected daily after 7 days of immunization. OA2
**Figure 3****:** Oleanolic acid diminished firm arrest (A) and rolling flux (B) of leukocytes on brain microvasculature, analyzed by intravital microscopy. C, healthy mice.
EAE, induced-mice. EAE+OA1, induced-mice treated with OA at the onset of the clinical signs. EAE+OA2, induced-mice treated with OA from day 7 after immunization.
**Figure 4****:** Changes in blood-brain barrier permeability of untreated- or OA treated EAE-mice. Evans blue dye was used as a measurement of plasma protein extravasation in (A) cerebral cortex, (B) cerebellum and (C) spinal cord 21-24 days post-immunization. C, healthy mice. EAE, induced-mice. EAE+OA1, induced-mice treated with OA at the onset of the clinical signs. EAE+OA2, induced-mice treated with OA from day 7 post-immunization.
**Figure 5****:** Effect of OA on osteopontin (OPN) expression in CNS tissues: (A) cerebral cortex, (B) cerebellum and (C) spinal cord, of healthy and EAE mice, analyzed by commercial ELISA kits. C, healthy mice. EAE, induced-mice. EAE+OA1, induced-mice treated with OA at the onset of the clinical signs. EAE+OA2, induced-mice treated with OA from day 7 post-immunization
**Figure 6****:** Survival enhancement of EAE mice due to effect of Oleanolic acid treatment, injected after first symptoms.

### BRIEF DESCRIPTION OF THE EMBODIMENTS

### Example 1.- Oleanolic acid reduces clinical signs in EAE mice.

In the experiments we used fifteen animals per group. EAE was induced as described (Slavin A. Autoimmunity 1998, 28: 109) in C57BL mice by administration of a proteolipid protein. The immunization was carried out with 100 µg of a partial peptide of myelin/oligodendrocyte glycoprotein (MOG₃₃₋₅₅) in complete Freund's adjuvant containing 4 mg of Mycobacterium tuberculosis H37Ra in 1 ml. The mice were immunized by subcutaneous injection of this emulsion on day 0. In addition, on day 0 and 2, were administered intraperitoneally 300 ng/200 µl of *Bordetella pertussis* toxin. The administration of 6 mg/kg of oleanolic acid was performed intraperitoneally once a day, beginning:
1 .- 12 days after induction of EAE, when clinical symptoms were detected , until the end of the experiment (21 days after induction) (OA1), and
2 .- after 7 days of induction of EAE, before the onset of the disease, until the end of the experiment (21 days after induction) (OA2).

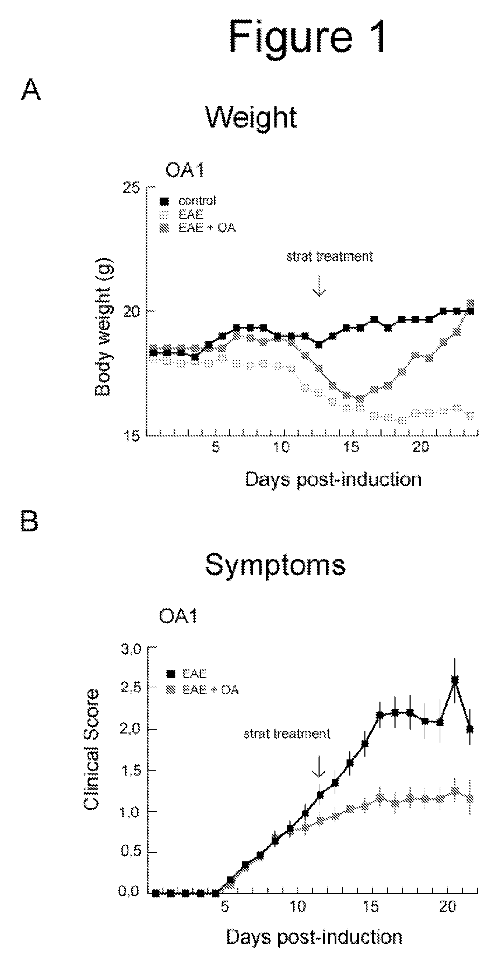

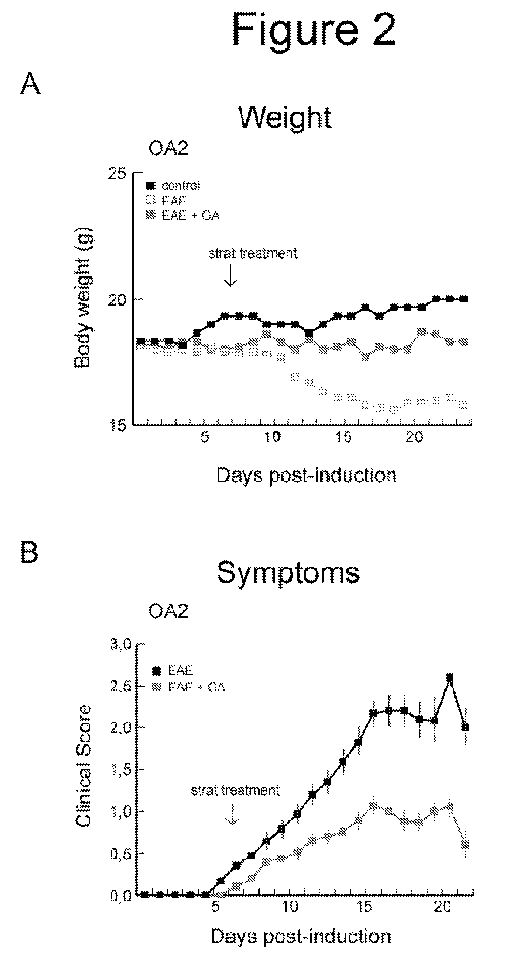

Mice were examined, weighed and scored daily in a double-blind manner for signs of EAE. OA-treated EAE mice were compared with a group of EAE animals treated with placebo, with untreated control animals or control animals received the same daily dose oleanolic acid. In addition, a pathologic evaluation was performed to confirm the effect of improving the state of the disease. The weight of the mice in each group is represented in Figure 1A and 2A. The level of paralysis reached by each group of mice is shown in Figures 1B and 2B.

### Evaluation of the effect of Oleanolic acid: Scores.

The effect of disease improvement (clinical effect) by the action of oleanolic acid is scored and evaluated with the pattern presented below.
Grade 0, no abnormality.
Grade 0.5, partial loss/reduced tail tone, assessed by inability to curl the distal end of the tail (Tail 50% or 2/3).
Grade 1.0, tail atony 100%
Grade 1,5, slightly/moderately clumsy gait, impaired righting ability, or combination.
Grade 2.0, hind limb weakness, partial (1 or 2)
Grade 2,5, partial (1 or 2) hind limb paralysis.
Grade 3, complete hind limb paralysis.
Grade 3,5, limb weakness.
Grade 4, tetraplegic. Complete limb paralysis
Grade 5, moribund state or death.
signs of paralysis started to develop around days 11-14 (tail atony and clumsy gait) following immunization, pointing to EAE induction;

In Figure 1B, it is shown the evolution of the signs of paralysis of untreated EAE mice, compared with OA treated EAE mice. Mice treated with 6 mg/kg of the natural triterpene compound isolated from olive pomace oil, oleanolic acid, showed a significant improvement in clinical status of the disease compared with untreated EAE group. In addition, the severity of EAE was also significantly attenuated with prophylactic administration of oleanolic acid, as there was a delay in the onset of the disease (Fig. 2B). In both situations, the reduction of body weight was suppressed, showing a recovery (Fig. 1A) or preventive (Fig. 2A) effect.

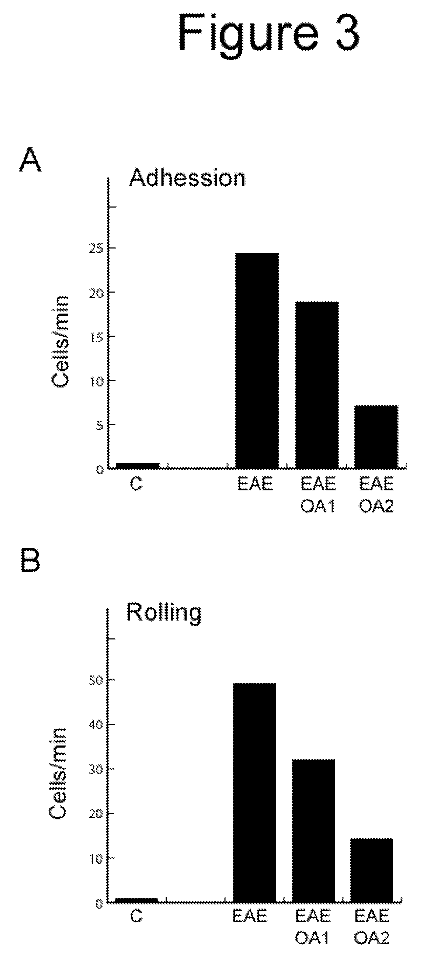

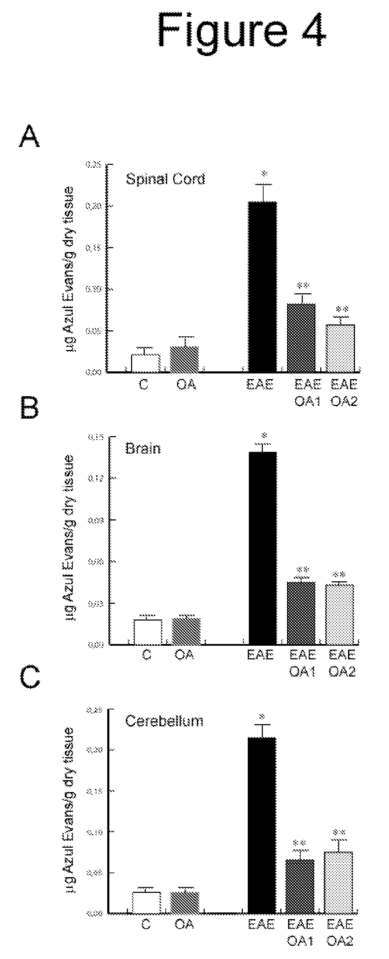

Moreover, as mentioned above, in MS there is an infiltration of inflammatory cells into the CNS because of the breakdown of the blood-brain barrier, causing both cellular and molecular extravasation. These phenomena occur mainly in the venules where blood flow is lower. To study leukocyte-endothelial interactions that occur *in vivo* in the microcirculation (particularly the phenomena of leukocyte rolling and adhesion), intravital fluorescence microscopy was used. Figure 3 shows the adherent (A) and rolling (B) leukocytes. EAE animals showed a significant increase of both parameters compared to healthy controls mice. In contrast, when the mice belonged to groups of protocol EAE+OA1 or protocol EAE+OA2, we observed a significant decrease in the flow of leukocytes rolling and adhering to the endothelium, compared with the untreated EAE group. Then, to characterize the changes in vascular permeability, Evans blue was injected intraperitoneally to the different groups of animals. Figure 4 shows the leakage that occurs in the spinal cord, brain and cerebellum. Mice of protocol EAE+OA1 and protocol EAE+OA2 have a significantly reduced leakage in the CNS tissues studied, as compared to untreated EAE animals.

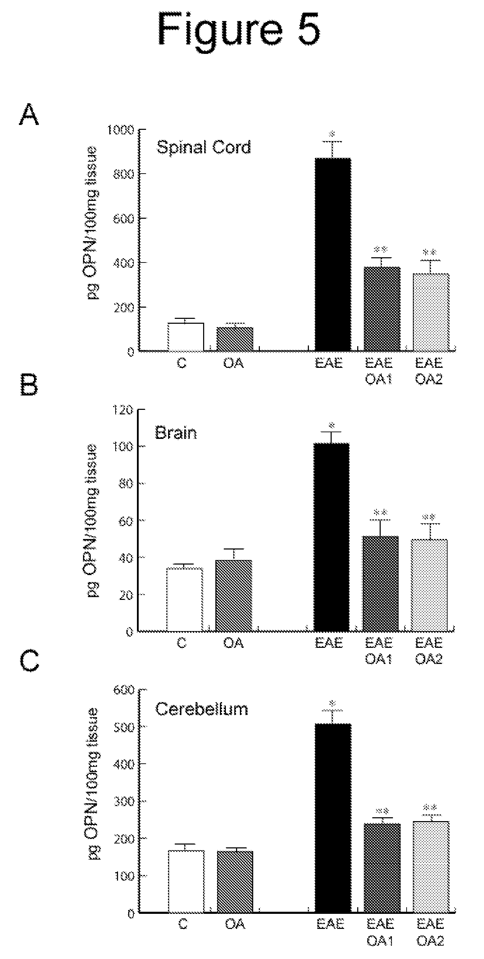

The analysis of key pro-inflammatory protein expression in EAE, lead us to study the presence and modulation of Osteopontin in CNS tissues. Figure 5 shows that in all tissues studied, spinal cord, brain and cerebellum, the expression of this protein is increased in mice with EAE, when compared with healthy control mice. This increase was significantly reduced when mice were treated with oleanolic acid, following either protocol OA1 or OA1.

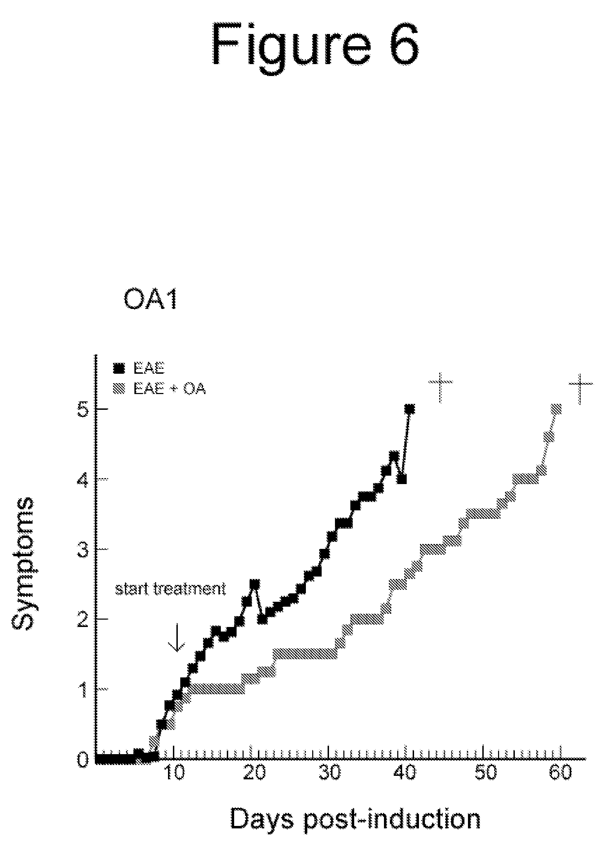

In terms of survival (Fig. 6), was noted that in the placebo-treated EAE group, mice die within the first 40 days after disease induction. In contrast, when EAE mice receive oleanolic acid at the onset of symptoms, the animals did not die until 60 days post-induction.

### Material and methods.

### Experimental animals.

Mice were housed in the animal care facility at the School of Medicine of the University of Valladolid. Mice were fed with a special diet for laboratory animals, water ad libitum, temperature of 20-24 ° C and exposed to a light cycle of 12 h/day (8.00 am - 8. 00 pm) (Council of European Communities, 1986). All experimental protocols were reviewed and approved by the Animal Ethics Committee of the School of Medicine, of the University of Valladolid.

### Reagents

The following chemicals used in the experiments were provided by Sigma Chemical Co. (St. Louis, MO, USA): Freud's complete adjuvant, M. tuberculosis H37 RA, B. pertussis toxin, rhodamine 6G and Evans Blue. The ELISA kit for detection of osteopontin was from IBL (Hamburg, Germany). The natural compound oleanolic acid was supplied by Cymit Química SL (Barcelona, Spain).

The pentacyclic triterpene, oleanolic acid was initially dissolved in dimethyl sulfoxide (DMSO) to prepare a stock solution of 10⁻² M. The subsequent dilutions of the triterpene were also carried out in DMSO. The final concentration of DMSO reached (less than 0.001 %) did not significantly affect the results.

### Induction of EAE

Chronic progressive EAE disease was induced in adult 8-10-wk-old female C57BL/J6 mice following the protocol described by (Slavin A. Autoimmunity 1998, 28:109-20). Mice were injected in the tail base bilaterally with an innoculum containing 100 µg of MOG peptide 35-55 emulsified in complete Freund's adjuvant containing 0.4 mg/ml Mycobacterium tuberculosis H37 RA. Then, i.p. injection 300 ng/animal of B. pertussis toxin was administered by two times with an interval of 48 hours. Mice were examined daily to monitor weight loss and the onset of neurological symptoms

### Analysis of osteopontin in the CNS.

Animals of the different experimental groups were sacrificed at day 21 post-immunization and CNS protein extracts (cerebral cortex, cerebellum and spinal cord) were prepared by homogenization in a solution: 0.4 M NaCl, 0.05% Tween 20, 0.5% BSA, 0.1 mM phenylmethylsulfonyl fluoride, 0.1 mM benzetonium chloride, 10 mM EDTA and 20 KI aprotinin (100 mg tissue / ml). The homogenate was centrifuged at 10,000 rpm for 10 min at 4 ° C. The concentration of osteopontin was determined in the supernatants by a commercial ELISA kit.

### Intravital microscopy in the brains of mice.

Intravital microscopy techniques allow the study of leukocyte-endothelium interactions. The leukocytes that interact with the endothelial surface can be visualized because their speed is markedly reduced, as compared to the average velocity of the blood flow in the venule. To make a precise study of leukocyte-endothelium interactions, we first made a record of the venule for one minute using a camcorder, and in a second time, a more detailed analysis was performed. The parameters of interest were the number of leukocytes adhering to the venular endothelium (number of leukocytes stationary for more than 30 seconds), and the number of rolling leukocytes (number of white cells per minute moving at a velocity less than that of erythrocytes).

The craniotomy was performed on the parietal zone. Animals received i.v. administration of rhodamine 6G (0,3 mg/kg body weight) to fix leucocytes. The fluorescence associated to rhodamine 6G was visualized at 510-560 nm by epi-ilumination. The microscope (20x) was used for observing micro-circulating events in brain. A digital camera coupled with a microscope displays the images on a monitor, which were recorded on video for further analysis of the leucocyte in rolling and adhesion.

### Analysis of extravasation alterations

Evans blue dye is able to bind quantitatively to albumin, both *in vivo* and *in vitro.* This property has been widely used for quantification of protein extravasation as an index of increased vascular permeability and, indirectly, of tissue damage. The Evans Blue, once extravasated into the tissues, is removed from them, and then quantified by visible spectrophotometry.

Mice from all experimental groups were given 30 mg/kg of Evans Blue i.p. Then, the spinal cord, cerebrum and cerebellum was removed and washed in saline. The dye was extracted from the CNS tissues with formamide and the concentration was determined by measuring the absorvance at 620 nm. CNS tissue was dried 24 h at 60°C and weighed. The extravasated dye was expressed as µg of Evans Blue/mg dried weight of the tissue.

### Statistical Analyses

Data were treated using Mann-Whitney U test. Results are expressed as mean ± SD; P values <0,05 were considered statistically significant.

## Claims

1. Oleanolic acid for use in the prevention and/or treatment of multiple sclerosis.

2. A pharmaceutical composition for use in the treatment of multiple sclerosis wherein the composition comprises a therapeutically effective amount of oleanolic acid, together with, optionally, one or more adjuvants and/or pharmaceutically acceptable vehicles.

3. The pharmaceutical composition for use according to claim 2 further comprising one or more additional therapeutic agents.

## Patentansprüche

1. Oleansäure zur Prävention und/oder Behandlung von multipler Sklerose.

2. Eine pharmazeutischen Zusammensetzung zur Behandlung von multipler Sklerose, wobei die Zusammensetzung eine therapeutisch wirksame Menge an Oleansäure zusammen mit wahlweise einem oder mehreren Hilfsstoffen und/oder pharmazeutisch annehmbaren Trägern umfasst.

3. Die pharmazeutische Zusammensetzung zur Verwendung nach Anspruch 2, die weiterhin einen oder mehrere zusätzliche therapeutische Wirkstoffe umfasst.

## Revendications

1. L'acide oléanolique à utiliser dans la prévention et/ou le traitement de la sclérose en plaques.

2. Une composition pharmaceutique à utiliser dans le traitement de la sclérose en plaques, dans laquelle la composition comprend une quantité thérapeutiquement efficace d'acide oléanolique, conjointement avec, éventuellement, un ou plusieurs adjuvants et/ou véhicules de qualité pharmaceutique.

3. La composition pharmaceutique à utiliser selon la revendication 2 comprenant en outre un ou plusieurs agents thérapeutiques supplémentaires.
